# EUROPEAN PATENT SPECIFICATION

(11) **EP 3 380 084 B1**
(45) Date of publication and mention of the grant of the patent: **21.08.2019**
(21) Application number: 17767768.9
(22) Date of filing: 05.09.2017
(51) Int. Cl.: A61K 9/16, A61K 31/4439

(54) **OMEPRAZOLE FORMULATIONS**
OMEPRAZOLFORMULIERUNGEN
FORMULATIONS D'OMÉPRAZOLE

(30) Priority: 07.09.2016 EP 16187705
(43) Date of publication of application: 03.10.2018
(73) Proprietor: Sandoz AG, 4056 Basel (CH)
(72) Inventor: STARIC, Rok, 1526 Ljubljana (SI); PETEK, Bostjan, 1526 Ljubljana (SI)
(74) Representative: Prüfer & Partner mbB Patentanwälte · Rechtsanwälte
(86) International application number: PCT/EP2017/072243
(87) International publication number: WO 2018/046498

(56) References cited:
- EP-A1- 0 519 870
- WO-A1-2010/041276

## Description

### Field of the invention

The present invention belongs to the field of pharmaceutical industry and relates to pellets comprising pharmaceutically active ingredient and binder. Further, the invention refers to a process for preparing said pellets, to the use of said pellets for preparing a pharmaceutical composition, and to a dosage form comprising said pellets or pharmaceutical composition. Finally, the present invention refers to a dosage from for use in a method of treating a disease selected from the group consisting of gastro esophageal reflux disease, peptic ulcer disease, and Zollinger-Ellison syndrome and for prevention of upper gastrointestinal bleeding in people who are at high risk.

### Description of the background art

The racemate of (*RS*)5-methoxy-2-[[(4-methoxy-3,5-dimethyl-2-pyridinyl)methyl]sulfinyl]-1H-benzimidazole is known as omeprazole and is described for instance in EP 0,005,129. Its R-enantiomer is known as esomeprazole. Also the S-enantiomer can be provided in pure form. Furthermore, pharmaceutically acceptable salts of the aforementioned compounds are known. Omeprazole is generally known to be useful for inhibiting gastric acid secretion in mammals and man by controlling gastric acid secretion at the final step of the acid secretory pathway and can be used in the treatment of gastric and duodenal ulcers. It is an inherent property of omeprazole to be activated to the active moiety in the acid environment within the parietal cells. The activated compound interacts with the enzyme in the parietal cells, which mediates the production of hydrochloric acid in the gastric mucosa. Omeprazole contains a sulfoxide grouping, which interferes with the H⁺K⁺-ATPase in the parietal cells hitherto known are all also degraded in acid media.

Omeprazole is a proton pump inhibitor (PPI) and a potent inhibitor of gastric acidity which is widely used in the therapy of gastroesophageal reflux and peptic ulcer disease. Omeprazole is an approved medicament for short-term treatment of duodenal and gastric ulcers, for the treatment of patients with symptomatic gastroesophageal reflux disease, for the treatment of erosive esophagitis and for the long-term treatment of pathological hypersecretory conditions (e.g., Zollinger-Ellison syndrome, multiple endocrine adenomas and systemic mastocytosis).

Omeprazole is however susceptible to degradation/transformation in acid reacting and neutral media. The half-life of omeprazole in water solutions at pH values less than 4 is shorter than 10 minutes. Also at neutral pH-values the degradation process proceeds rapidly, and at pH of about 7 the half-life of omeprazole is about 14 hours, while at higher pH-values the stability in solution is much better (Pilbrant and Cederberg, Scand. J. Gastroenterology 1985; 20 (supp. 108) p. 113-120). The stability profile is similar in solid phase. The degradation of omeprazole is catalyzed by acidic reacting compounds and is stabilized in mixtures with alkaline reacting compounds. The stability of omeprazole is also affected by moisture and organic solvents. Therefore, if omeprazole is administered orally, it must be protected from the gastric juices, which are acid, so that it may reach the small intestine where it is absorbed, in unaltered state (EP 0,247,983).

In human pharmacological studies it was found that the release rate of omeprazole from a pharmaceutical dosage form can influence the total extent of absorption of omeprazole to the general circulation (Pilbrant and Cederberg, Scand. J. Gastroenterology 1985; 20 (suppl. 108) p. 113-120). A fully bioavailable dosage form of omeprazole must release the active drug rapidly in the proximal part of the gastrointestinal canal.

In order to obtain a pharmaceutical dosage form of omeprazole which prevents omeprazole from contact with acidic gastric juice after oral intake, the pharmaceutical dosage form of acid labile substances must be enteric coated. Enteric polymers do not dissolve at low pH and by this protection of the drug is ensured. After the pH increase in small intestine the polymer dissolves and releases the drug. For safety and efficacy of patients it is essential that no release of omeprazole is achieved in acid environment in order to protect degradation of omeprazole. At higher pH rapid and fast release is required in order to ensure fast absorption of omeprazole and consequently pharmacological effect.

Enteric formulation is more preferred to be in form of multi-unit dosage system due to reduced risk of dose dumping and facilitated stomach passage due to smaller size of dosage form. The most common dosage form is a capsule which is filled with approximately 1 mm big particles called pellets. Pellets are usually filled into hard gelatin capsules. One of the possibilities to produce such pellets is by applying layers of pharmaceutically active ingredient and excipients onto neutral cores.

Pellets comprising neutral core covered by the active layer comprising omeprazole or one of its single enantiomers, optionally mixed with alkaline reacting compounds are disclosed in several prior art documents. For example WO 93/25204 discloses a stable microgranule formulation of omeprazole comprising an neutral core consisting of sugar and starch covered with an active layer constituted by omeprazole diluted in mannitol in substantially equal amounts, and an intermediate layer comprising mannitol, an outer layer formed from an enteric coating being optionally present. WO 98/052564 discloses a pharmaceutical composition which is a solid pellet comprising an neutral core, a benzimidazole in or on the core, preferably present in an alkaline environment, a moisture resistant coating around the core, the moisture resistant coating comprising at least one hydrophobic material, and an enteric coating around the moisture resistant coating. WO 96/23500 discloses an oral pharmaceutical formulation containing omeprazole which comprises a nucleus formed by an neutral core onto which an active layer containing omeprazole, non-alkaline reacting pharmaceutical excipient (e.g. talc) and water soluble neutral polymer (e.g. hydroxypropylmethycellulose, HPMC) is sprayed. WO 2010/041276 discloses esomeprazole pellets having an active to binder ratio in the active layer of between 3:1 to 5:1.

There are two main technologies for the production of pellets comprising an neutral core covered by the layer comprising pharmaceutically active ingredient. The first technology is a technology comprising wet granulation / extrusion / spheronization process, the method wherein after granulation by extrusion the neutral cores are made spherical e.g. with a marumerizer. The neutral cores so produced are most often not of an approximately spherical shape, and the granule size distribution is wide and therefore the uniform coating is difficult to achieve. In addition, carrying out the wet granulation / extrusion / spheronization manufacturing process normally requires the transfer of neutral cores from extruder/ spherionizer to the coating pans or fluid bed equipment in order to be coated. It also requires various equipment of restricted use and a high costs which is reflected in the final price of product.

The second technology is a drug layering process by using spraying of neutral cores with suspension or solution comprising pharmaceutically active ingredient in fluidized bed spray granulator. The layering process usually begins with neutral spherical cores that provide the solid surface on which the active layer comprising pharmaceutically active ingredient (also referred to herein as active agent, API or drug) is sprayed. Neutral cores are traditionally formed from pharmaceutically acceptable substances, such as lactose, sucrose, and starch. In prior art wherein omeprazole formulations containing pellets that comprise an neutral core covered by the active layer comprising omeprazole, the drug loading of less than about 15 % of omeprazole was used and the usual ratio between omeprazole and neutral cores of more than 1:4 was identified. For example in WO 93/25204 the drug loading of less than 10 % of omeprazole in final pharmaceutical formulation and the ratio between omeprazole and neutral cores of 1:4 is disclosed; in WO 96/23500 the drug loading of 6.6 % of omeprazole in final pharmaceutical formulation and the ratio between omeprazole and neutral cores of 1:7 is disclosed; in WO 98/052564 the drug loading of 3.6 % of omeprazole in final pharmaceutical formulation and high ratio between omeprazole and neutral cores (more than 1 : 4.75) is disclosed; in EP 1,108,425 the drug loading of less than 14 % of omeprazole in final pharmaceutical formulation and the ratio between omeprazole and neutral cores of more than 1:3.6 is disclosed; in WO 04/014345 the drug loading of 6.1 % of omeprazole in final pharmaceutical formulation and the ratio between omeprazole and neutral cores of 1 : 2.7 is disclosed.

By taking in-house SEM (Scanning electron microscope) pictures of commercially available omeprazole products from manufactures Kreamers, Zydus and Dr. Reddy the thickness of omeprazole layers in final pellets of approximately less than or about 40 µm was revealed. Further by the in-house testing it was revealed that the thickness of omeprazole layer in final pellets of approximately less than or about 40 µm corresponds to a drug loading of less than 10 % of omeprazole in final pellets.

In the initial phase of the process of fluid bed coating, there occur frequently troubles such as breaking and scraping of the neutral spherical cores, the moisture sensitivity of neutral cores (also referred to herein as neutral sugar spheres, or cores) causing problems with controlling the agglomeration and sticking, increased solubility and undesirable agglomerates formation of neutral cores. All of the above have great influence on the yield in production of pellets.

In EP 0,277,741 the problem of a breaking and scraping of the neutral spherical cores was solved by using simultaneous spraying of neutral cores with an aqueous binder and with spraying powder containing benzimidazole compound and low substituted hydroxypropylcellulose (HPC).

In EP 1,108,425 the fluid bed equipment with an inner partition device (wurster) and the manufacturing process without using organic solvents in any of the phase of technological process is described as preferred, cheaper and environmental friendly option for producing pellet formulations composed of an neutral core coated with a drug containing layer, coated in turn with an intermediate layer and with a final gastroresistant or enteric layer.

There is a strong competition on omeprazole market and therefore there is a high need and thus an object for improved pharmaceutically active ingredient containing pellets, and for an improved method preparing said pellets, and for dosage forms comprising said pellets.

### Summary of the invention

The scope of the invention is defined by the claims:
1. Pellet comprising
   - a neutral core comprising one or more pharmaceutically acceptable excipients;
   - a pharmaceutically active ingredient layer surrounding the neutral core, wherein said pharmaceutically active ingredient layer comprises omeprazole, one or more binders and optionally one or more further excipients; wherein the thickness of said pharmaceutically active ingredient layer is more than 50 µm at one or more positions; and wherein the weight ratio of omeprazole:binder(s) is in the range of from 3:1 to 5:1; and
   - wherein the the weight ratio of omeprazole:neutral core is in the range of from 1:1.5 to 1:4.

The "pharmaceutically active ingredient layer" is also called "drug-containing layer", "active layer", or "API-containing layer" and means a layer comprising omeprazole.

The core is an inert core.

In a further preferred embodiment, the pellet comprises, preferably consists of, the neutral core, the pharmaceutically active ingredient layer, the protective layer, and the enteric coating, with the coatings and layers as respectively disclosed elsewhere herein.

### Detailed description of the invention

Within the context of the present invention, it has now unexpectedly been found that a pellet comprising a neutral core comprising one or more pharmaceutically acceptable excipients, a pharmaceutically active ingredient layer surrounding the core, wherein said pharmaceutically active ingredient layer comprises omeprazole; one or more binders; and optionally one or more further excipients, wherein the pharmaceutically active ingredient layer has a certain, specific thickness and a certain, specific weight ratio of the pharmaceutically active ingredient to binder(s), and wherein the pellet optionally exhibits a certain, specific relative content of pharmaceutically active ingredient based on the total weight of the pellet, and exhibits improved properties e.g. with regard to dissolution and/or handling. Moreover, the manufacturing process of such pellets, pharmaceutical compositions and/or final dosage forms such as capsules can be improved, for instance with regard to time, costs, and/or batch size.

The present invention can offer an efficient solution for improved manufacturing of omeprazole-comprising pellets and/or pharmaceutical compositions and/or final dosage forms, without the need for upgrading the manufacturing facility with new manufacturing equipment. In the present invention, it has been surprisingly found that manufacturing efficiency can be significantly improved by carefully selecting the weight ratios of pharmaceutically active ingredient (API) to binders, the thickness of the pharmaceutically active ingredient layer (also referred to herein as drug-containing layer, active layer, active agent layer or API layer), and a minimum content of pharmaceutically active ingredient in the pellet based on the total weight of the pellet.

By using the pellets of the present invention, for instance the amount of needed excipients for producing pharmaceutical compositions (such as dosage forms comprising the pharmaceutically active ingredient of the present invention) can be reduced, the size of production batch (namely the number of final dosage forms such as capsules per production batch) can be increased, and/or the production time can be decreased. As the omeprazole market is increasing in the amount of needed final dosage forms (such as capsules), the increase of dosage form number per batch on same equipment is of great importance for the industry.

Comparably high relative loading of pharmaceutically active ingredient and comparably increased thickness of pharmaceutically active ingredient layer, without the pellets being too sticky for proper manufacturing at the same time, was achieved by selection of appropriate ratio between binder and pharmaceutically active ingredient.

The high loading of pharmaceutically active ingredient relative to the neutral pellet weight results in smaller final dosage forms, preferably capsule, comprising pharmacologically efficient amounts of high relative loading of pharmaceutically active ingredients. What would increase a patient compliance.

Further, to overcome the dissolution limitation due to the increased thickness of the pharmaceutically active ingredient layer, it has surprisingly been found that increasing binder content until a certain weight ratio of pharmaceutically active ingredient:binder is reached, can enhance the dissolution rate from pharmaceutical composition comprising a neutral core covered by a pharmaceutically active ingredient layer, despite the general expectation that increasing binder amount would slow down the dissolution.

The pharmaceutically active ingredient used in the present invention is omeprazole, which is a drug used in the treatment of e.g. peptic ulcer disease, and Zollinger-Ellison syndrome. It is also used to prevent upper gastrointestinal bleeding in people who are at high risk.

Omeprazole and esomeprazole are highly sensitive to acid environment of stomach. For this reason medication, final dosage forms or pharmaceutical compositions comprising omeprazole or esomeprazole need to be protected against stomach acid by means of coating the pharmaceutically active ingredient with enteric polymer. Enteric polymers do not dissolve at low pH and by this protecting the pharmaceutically active ingredient. After the pH increases in small intestine, the enteric polymer dissolves and releases the pharmaceutically active ingredient. For safety and efficacy of patients it is desired and thus aimed at that essentially no release of omeprazole or esomeprazole is achieved in acid environment in order to prevent degradation. At higher pH, a rapid and fast release of omeprazole or esomeprazole is required in order to ensure fast absorption of omeprazole or esomeprazole and consequently pharmacological effect. It is preferred that such enteric formulation is in the form of a multi-unit dosage system, due to a reduced risk of dose dumping and facilitated stomach passage due to a smaller size of the dosage form. The most common dosage form is capsule which is filled with pellets. One of the possibilities to produce such pellets is by applying layers of pharmaceutically active ingredient and excipients onto a neutral sugar spheres or cores.

However, for instance when aiming to increase batch size, the amount of neutral cores could be increased. Such an increased quantity of neutral cores can cause several disadvantages during the manufacturing process, e.g. i) lower drug loading and consequently decreased batch size, longer production time and increased amount of needed excipients; ii) loss of yield due to the friability of neutral cores and therefore increased expense and difficulty of manufacture.

The solution to one or more, preferably all, of the above problems is the reduction of the amount of neutral cores on which omeprazole is positioned, preferably layered, and increasing binder amount in the pharmaceutically active ingredient layer. By reducing the amount of neutral cores the dissolution profile was reduced and it was surprisingly found that the problem can be solved by providing pellets according to the present invention, namely pellets that exhibit a certain minimum thickness of pharmaceutically active ingredient layer and that comprises a specific, weight ratio of pharmaceutically active ingredients:binders

The pellets according to the present invention can provide higher pharmaceutically active ingredient loading; reduced amount of needed excipients, increased batch size, reduced production time, smaller size of final dosage form, preferably capsule, improved manufacture efficacy, decreased energy consumption during the manufacturing process, and/or positive impact on environment.

Omeprazole products that are at present commercially available usually revealed a thickness of the pharmaceutically active ingredient layer of up to approximately 40 µm.

By selecting appropriate excipients and layering technology it has surprisingly been found that there is a possibility of providing pellets that comprise comparably higher pharmaceutically active ingredient loading, and that it is additionally possible to increase the thickness of the pharmaceutically active ingredient layer, e.g. to approximately 100 µm or even up to approximately 150 µm. This was achieved by reducing the amount of neutral cores onto which the active agent is positioned, preferably layered.

Additionally, in usual active ingredient layering technologies, a higher ratio of pharmaceutically active ingredient:neutral core is used (for example pharmaceutically active ingredient:neutral core = 1:5 or more). By applying the present invention, this ratio can be reduced even to e.g. 1:2. In other words, by applying the teaching of the present invention, the amount of the pharmaceutically active ingredient per pellet can be increased compared to prior art pellets, the thickness of the pharmaceutically active ingredient layer is increased compared to prior art pellets, and at the same time the stickiness of the pellets can be prevented.

As disclosed elsewhere herein, an important role in achieving such high pharmaceutically active ingredient loading is the selection of an appropriate binder amount, or weight ratio of pharmaceutically active ingredient:binder, respectively. The binder role is to stick particles of pharmaceutically active ingredient on neutral cores during manufacturing, e.g. during layering process. In the present invention it has been shown that increasing binder:omeprazole ratio to a ratio of at least 2:10 is sufficient to achieve appropriate binding of omeprazole onto neutral cores.

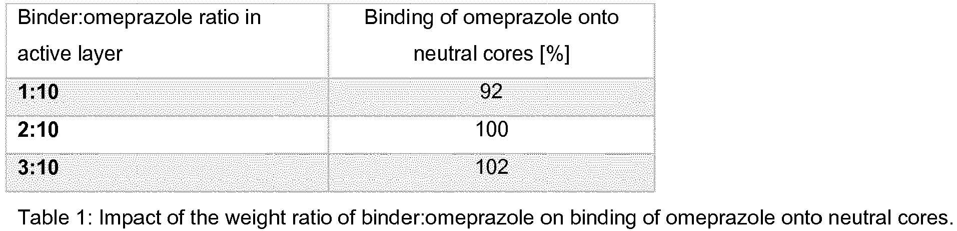

Even though the ratio of binder:omeprazole being 2:10 is sufficient for the binding of the active ingredient onto the neutral core during manufacturing process, in the context of the present invention surprising benefits were obtained additionally by increasing the binder concentration.

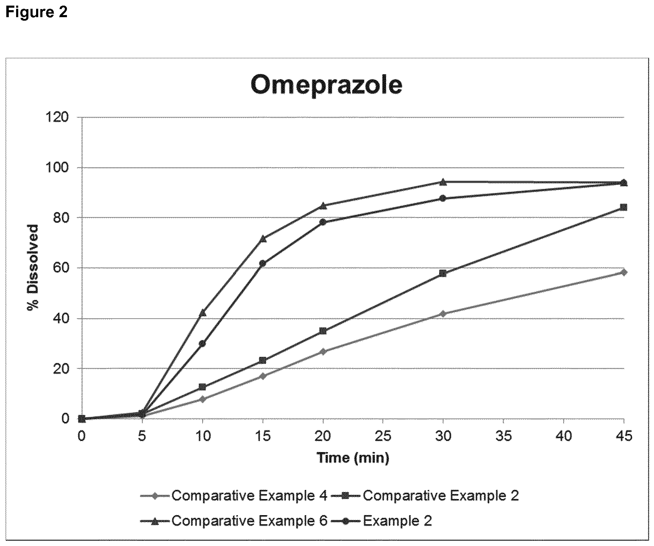

As the thickness of pharmaceutically active ingredient layer (also referred to herein as drug loading) is increased due to the lower amount of neutral cores, the dissolution rate of pharmaceutically active ingredient is decreased, as can be seen when comparing the dissolution rate of Comparative example 4 with the dissolution rate of Comparative example 2 (Fig. 2). This can be contributed to the increased thickness of the pharmaceutically active ingredient layer for comparative example 4 (Fig. 2) which is dissolution limiting factor.

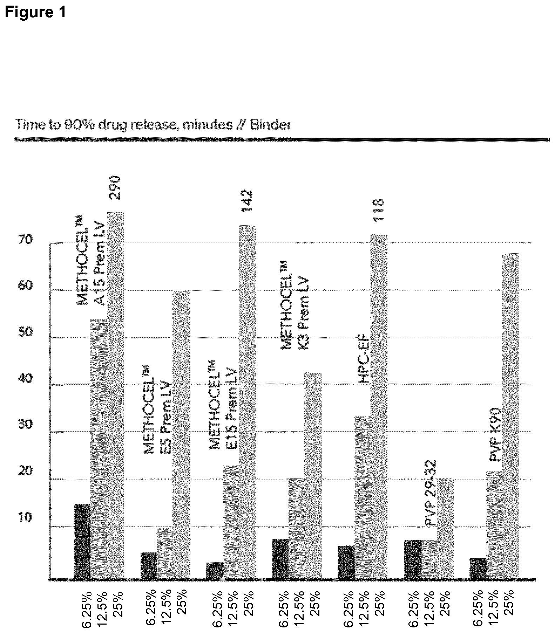

We surprisingly found that increasing the amount of binder enhances dissolution rate from enteric coated pellets. If the amount of binder was high, the dissolution rate was increased, as can be seen when comparing the dissolution rate of Example 2 with the dissolution rate of Comparative example 4 (Fig. 2). As it is generally accepted that increasing binder amount would slow down the dissolution (Fig. 1), this was a surprising effect of the binder.

At the same time, however, it has surprisingly been found in the context of the present invention that the advantageous effects of the present invention can only be achieved if a certain, defined range of weight ratio of pharmaceutically active ingredient :binder is fulfilled. If the content of binder, relatively to the pharmaceutically active ingredient API content, is too high, then disadvantageous properties of the pellet occur. An example of such a disadvantageous property is increased stickiness of the pellets, which can result in severe problems during manufacturing. If the content of binder is too low, then there can occur difficulties with loading the desired high amount of pharmaceutically active ingredient onto a neutral core.

Hence, within the context of the present invention it has now unexpectedly been found that when applying a certain high amount of pharmaceutically active ingredient, and the thickness of the pharmaceutically active ingredient layer being at least 50 µm at one or more positions, then a certain defined range of weight ratio of pharmaceutically active ingredient(s):binders has to be fulfilled, in order to arrive at pellets that exhibit improved properties.

The present invention thus relates to a pellet comprising a neutral core comprising one or more pharmaceutically acceptable excipients, wherein a pharmaceutically active ingredient layer surrounds the core, wherein said pharmaceutically active ingredient layer comprises omeprazole; one or more binders; and optionally one or more further excipients. Additionally, the thickness of said pharmaceutically active ingredient layer is at least 50 µm at one or more positions, the weight ratio of pharmaceutically active ingredient:binders is in the range of from 3:1 to 5:1, preferably 3.5:1 to 5:1, more preferably 3.5:1 to 4.5:1, even more preferably 4:1 to 4.5:1.

The active agent is omeprazole. If the pharmaceutically active ingredients consist of a mixture of (S)- and (R)-form, then it is preferred that 40-60% of the pharmaceutically active ingredients are of (S)-form.

Within the context of the present invention it has been found that pellets can be provided that exhibit a comparably high drug loading, i.e. the content of pharmaceutically active ingredients in the pellet is 20 to 50 wt.-%, preferably 20 to 40 wt.-%, more preferably 25 to 35 wt.-%, even more preferably 25 to 30 wt.-%.

The weight ratio of pharmaceutically active ingredients:neutral core is in the range of from 1:1.5 to 1:4, preferably is within the range of from 1:1.5 to 1:3, more preferably from 1:1.8 to 1:2.5, and even more preferably from 1:2 to 1:2.5.

A high pharmaceutically active ingredient content in pellets of the present invention is in line with an increased thickness of the pharmaceutically active ingredient layer of at least 50 µm at one or more positions. In preferred embodiments, the thickness of the pharmaceutically active ingredient layer is in a range of from 60 µm to 150 µm, more preferably from 70 µm to 140 µm, and even more preferably from 80 µm to 120 µm at one or more positions. The thickness of any layer that is present in the pellet according to the present invention can be determined by any suitable means that is known to a person skilled in the art. An example of such a suitable means is scanning electron microscopy (SEM).

Within the meaning of the present invention, the term "core" defines a neutral (inert) starter material for pellet preparation and in general encompasses spheres, seeds, pellets, spheroids, granules, beads, particles, and the like. The neutral core that is used for being coated with the pharmaceutically active ingredient layer can be any suitable neutral core that is known to a person skilled in the art, having any suitable size. In a preferred embodiment, the neutral core comprises one or more pharmaceutically acceptable excipients that are selected from the group consisting of inorganic salts, metal oxides, organic polymers, such as celluloses, starches and sugars. In a preferred embodiment, the neutral core is a neutral sugar core. In a further preferred embodiment, the neutral core comprises sugar and/or maize starch.

In a further preferred embodiment, the core is a neutral sugar sphere comprised of 80.0 - 91.5 wt.-% sucrose and 8.5 - 20 wt.-% maize starch.

In an embodiment according to the present invention, the neutral core can have any suitable size. It is however preferred that the neutral core has a mean particle size of from about 250 µm to about 1000 µm, preferably from about 400 to about 900 µm, more preferably from about 600 to about 850 µm, even more preferably from about 700 to about 800 µm as determined by sieve analysis. Additionally, it is preferred that the neutral core has approximately spherical shape. The shape of the neutral core can be assessed by any suitable method that is known to a person skilled in the art, preferably the shape is assessed by applying SEM. Within the meaning of the present invention, the term "approximately spherical shape" denotes that the neutral core has a certain diameter, with a deviation of +/-20 %.

If the neutral core is not in a sufficiently spherical shape, then suitable measures have to be taken or making the neutral cores sufficiently spherical. Such measures are known to a person skilled in the art and comprise for instance the use of analysis marumerizer. An approximately spherical shape allows for a more uniform coating of the neutral core with the subsequent layers such as the pharmaceutically active ingredient layer, therefore providing pellets that are improved e.g. with regard to content uniformity, or overall handling.

It is further preferred that the neutral core is inert.

On the neutral core, a pharmaceutically active ingredient layer that surrounds the neutral core is present. It is possible that there are one or more additional layers between the neutral core and the pharmaceutically active ingredient layer. The one or more additional layers that can be present between the core and the pharmaceutically active ingredient layer as disclosed herein can for instance be layer that comprise additional pharmaceutically active ingredient, such as acid-labile pharmaceutically active ingredient, that is not omeprazole.

It is further possible that the layers are coatings that protect the neutral core from mechanical pressure during manufacturing process. It is however preferred that the pharmaceutically active ingredient layer is placed directly on the neutral core, i.e. that there is no further layer between the neutral core and said pharmaceutically active ingredient layer.

The pharmaceutically active ingredient layer comprises, beside omeprazole, one or more binders, and optionally one or more further excipients.

Binders act as an adhesive to bind together the ingredients of e.g. pharmaceutical compositions to result in the necessary mechanical strength. In general, a person skilled in the art knows which binders are suitable for a certain application or pharmaceutical composition. Preferred binders that are present in the drug-containing layer of the present invention are selected from the group consisting of celluloses, such as hydroxypropyl methylcellulose (HPMC; Hypromellose), hydroxypropyl cellulose (HPC) and carboxymethyl-cellulose sodium, polyvinyl pyrrolidone (PVP); sugars; and starches. In a preferred embodiment, the binder is hydroxypropyl methylcellulose (HPMC).

The content of pharmaceutically active ingredients in the pellet according to the present invention is at least 15 wt.-%, preferably at least 20 wt.-% t based on the total weight of the pellet. Thus, in a preferred embodiment, the pharmaceutically active ingredient layer comprises at least 80 wt.-%, preferably 85 wt.-%, more preferably at least 90 wt.-% of pharmaceutically active ingredients and binder together. It is additionally preferred that the binder content in the drug-containing layer is at least 15 wt.-%, and/or that the pharmaceutically active ingredient content in the drug-containing layer is at least 65 wt.-%.

Optionally, one or more further excipients, in addition to the pharmaceutically active ingredients and binder, can be present in the drug-containing layer.

The pharmaceutically active ingredient layer optionally comprises one or more further excipients selected from the group consisting of an alkaline reacting compound and one or more surfactants. An alkaline reacting compound is a compound that is able to neutralize acidic compounds. Acidic compounds directly or indirectly interact with pharmaceutically active ingredient that is acid-labile, such as omeprazole, and can thus for instance lead to a degradation of acid-labile compounds. Accordingly, the presence of an alkaline reacting compound is advantageous if there is the possibility that an acid-labile pharmaceutically active ingredient comes into contact with acidic compounds or with an acidic environment, as the alkalizing agent stabilizes the pharmaceutically active ingredient comprising.

In a preferred embodiment, the alkaline reacting compound is selected from the group consisting of sodium, potassium, calcium, magnesium and aluminum salts of phosphoric acid; carbonic acid; citric acid or other weak inorganic or organic acids; aluminum, calcium and magnesium hydroxides; magnesium oxide or composite substances, such as Al₂O₃·6MgO·CO₂·12H₂O, (Mg₆Al₂(OH)16CO₃·4H₂O), MgO·Al₂O₃·2SiO₂·nH₂O and organic pH-buffering substances such as trihydroxymethylaminomethane. Preferably, the alkaline reacting substance is magnesium oxide.

In addition to the binder, the pharmaceutically active ingredient layer can additionally comprise one or more surfactants. Surfactants are also called surface-active agents or solubilizing agents, used to disperse pharmaceutically active ingredient in a particular solvent and also enhance wetting properties of the pharmaceutically active ingredient thereby helps either to lower the surface tension of a liquid, allowing easier spreading, and lower the interfacial tension between two liquids or to solubilize the pharmaceutically active ingredient either in composition or *in-situ* at the site of absorption or action. They contain both hydrophobic groups and hydrophilic groups, thus being soluble in both organic solvents and water.

In a preferred embodiment, the surfactant is selected from pharmaceutically acceptable nonionic or ionic surfactants, such as sodium lauryl sulfate or polysorbate. In a preferred embodiment, the surfactant is sodium lauryl sulfate.

It is possible that the pellet of the present invention does not comprise an enteric layer and/or a protective layer. In one embodiment, the pellet does neither comprise an enteric layer nor a protective layer. In this case, the pellet only consists of a neutral core and a pharmaceutically active ingredient layer as disclosed herein. Such a pellet represents an intermediate product. In a preferred embodiment, the intermediate pellet consists of a neutral core, and a drug-containing layer, with the following composition:
(i) 50 - 70 wt.-%, preferably 55 - 65 wt.-%, more preferably 60 - 65 wt.-% neutral core, and,
(ii) 20 - 40 wt.-%, preferably 25 - 35 wt.-percent, more preferably 25 - 30 wt.-% omeprazole,
   5 - 8 wt.-%, preferably 6 - 7 wt.-% binder, preferably hydroxypropyl methylcellulose,
   0.5 - 2.0 wt.-%, preferably 0.5 - 1.5 wt.-% alkaline reacting compound, preferably magnesium oxide,
   0.5 - 2.0 wt.-%, preferably 0.5 - 1.5 wt.-% surfactant, preferably sodium lauryl sulfate,
   all under (ii) included in a pharmaceutically active ingredient layer.

In an even more preferred embodiment, the pellet comprises a neutral core and a pharmaceutically active ingredient layer with the following composition:
(i) 50 - 70 wt.-%, preferably 55 - 65 wt.-%, more preferably 60 - 65 wt.-% a neutral sugar sphere, and
(ii) 20 - 40 wt.-%, preferably 25 - 35 wt.-%, more preferably 25 - 30 wt.-% omeprazole,
   5 - 8 wt.-%, preferably 6 - 7 wt.-% hydroxypropyl methylcellulose,
   0.5 - 2.0 wt.-%, preferably 0.5 - 1.5 wt.-% magnesium oxide,
   0.5 - 2.0 wt.-%, preferably 0.5 - 1.5 wt.-% sodium lauryl sulfate,
   all under (ii) included in a pharmaceutically active ingredient layer.

The pharmaceutically active ingredient layer of the pellet of the present invention can be prepared according to any suitable method or process that is known to a person skilled in the art. In a preferred embodiment, the pharmaceutically active ingredient layer is applied onto the preceding layer or the neutral core, respectively, by a layering process using a fluidized bed spray granulator (e.g. Huettlin HKC 200), wherein a suspension or solution comprising the one or more pharmaceutically active ingredient, the binder and the optional further ingredients is sprayed onto the neutral core and dried. It is preferred that the pharmaceutically active ingredient layer is applied by using a fluidized bed granulator.

For instance, the one or more pharmaceutically active ingredient comprising in the respective desired amount, for instance 10mg, 20mg, or 40mg, the binder, and if present the surfactant and if present the alkalizing agent are dispersed in water by using a suitable mixer such as a propeller mixer. The neutral cores (e.g. the neutral sugar cores) are loaded into an appropriate fluid bed apparatus (e.g. Huettlin HKC 200). The neutral cores are heated to an appropriate temperature (e.g. 40 °C) for instance by using hot air, and then they are coated with a prepared dispersion comprising the one or more pharmaceutically active ingredient (omeprazole). Finally, the coated pellets are sieved in order to remove any fines or agglomerates that may be present.

As already disclosed elsewhere, the pellets of the present invention exhibit a reduced portion of agglomerates.

The present invention also refers to the pellets of the present invention, wherein the pharmaceutically active ingredient layer is obtainable or obtained by spraying a suspension or solution comprising the ingredients of the pharmaceutically active ingredient layer onto the neutral core and performing a drying process. In a preferred embodiment, the pharmaceutically active ingredient layer is prepared in fluidized bed granulator.

The present invention also refers to a pellet as disclosed herein, which is obtainable or obtained by a layering process by using a fluidized bed spray granulator.

It is possible that the pellet comprises one or more protective layer(s) above (e.g. placed upon, preferably directly placed upon) the pharmaceutically active ingredient layer (from the inside to the outside). Optionally, there can be an enteric coating above the one or more protective layers.

The protective layer (or protective coating) is generally applied onto the pharmaceutically active ingredient layer to separate the layer containing the pharmaceutically active ingredient (omeprazole) from an enteric coating layer (also referred to herein as enteric layer). Enteric layers usually comprise gastro-resistant polymers that have free carboxyl groups with acidic characteristics. If acid-labile pharmaceutically active ingredients such as the pharmaceutically active ingredient of the present invention come into contact with these enteric polymers, this could negatively influence the acid-labile pharmaceutically active ingredient such as causing the degradation of said pharmaceutically active ingredient. For this reason, it is preferred that a protective layer is present in order to separate the pharmaceutically active ingredient layer from the enteric layer.

The protective layer can be of any suitable material that is known to a person skilled in the art that is able to protect the active agent from negative influence (such as degradation) from further layers that may be present in the pellet, in particular from enteric layer(s). In a preferred embodiment, the protective layer is a water soluble or in water rapidly disintegrating layer, or a polymeric, water soluble, film-coating layer, optionally containing pH-buffering, alkaline compounds. In an even further preferred embodiment, the protective layer comprises povidone (polyvinylpyrrolidone) and talc.

Enteric coatings are known in the art and are typically designed to not/not substantially dissolve in the stomach. An example of commercially available enteric coating polymer is known under the tradename EUDRAGIT® (Evonik, Germany). For instance, EUDRAGIT®S 100 dissolves at a pH value above 7.0 which corresponds to the conditions as present in the colon. This leads to the effect that the enteric coating does not dissolve in the stomach, but in the colon. A further example of an enteric coating polymer is EUDRAGIT® L100-55 or L30D-55, which dissolve at a pH value above 5.5, which means that it dissolves in the duodenum. Further enteric coating examples and further enteric coating polymers exist.

Preferably the enteric polymer/enteric coating fulfils the requirements for enteric coatings as defined in the European Pharmacopoeia, chapter 2.9.3. (7^{th} Edition 2011 (7.2)).

In one embodiment, the enteric polymer is an anionic polymer or copolymer, preferably at least one selected from the group consisting of enteric cellulose derivatives, enteric acrylic copolymers, enteric maleic copolymers, enteric polyvinyl derivatives and shellac. Even further preferred, the enteric polymer is an anionic polymer with methacrylic acid as a functional group. Further preferred the enteric polymer is an anionic copolymer selected from the group consisting of methacrylic acid/ethylacrylate, methacrylic acid/methyl methacrylate, and methacrylic acid/methylacrylate/methyl methacrylate.

In one embodiment, the anionic polymer or mixture of anionic polymers is used in combination with an methacrylate copolymer having neutral ester groups (e.g. R=COOCH₃ or R=COOC₄H₉) or trimethylammonioethyl groups (R=COOCH₂CH₂N⁺(CH₃)₃Cl). Methacrylate copolymers having a neutral ester are insoluble and pH-independent and can be used to provide a time controlled release dosage form, wherein e.g. after dissolution of the anionic polymer a matrix of the insoluble polymer remains and the medicinal substance can be washed out of this matrix.

In a preferred embodiment, the enteric polymer/enteric coating layers dissolve(s) above a pH of 5.5. A dosage form comprising such enteric coating layers will therefore release the pharmaceutically active ingredient directly after leaving the stomach, e.g. in the duodenum. In another embodiment of the invention, the enteric polymer/enteric coating layers dissolve(s) at a pH of between 5.5 and 7.0.

In another embodiment, the enteric polymer/coating layers dissolve(s) at a pH above 7.0. In another embodiment, the enteric polymer/enteric coating layers dissolve(s) at a pH of between 5.5 and 6.5, most preferred at a pH of between 5.5 and 6.0.

Preferred enteric cellulose derivatives according to the present invention are hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose or ethylhydroxyethylcellulose phthalate.

Suitable hydroxymethylethylcellulose phthalates are also sold unter the tradename HP50® and HP55® (Shin-Etsu, Japan).

Preferred enteric acrylic copolymers are styrene/acrylic acid copolymer, methyl acrylate/ acrylic acid copolymer, methyl acrylate/ methacrylic acid copolymer, butyl acrylate/ styrene / acrylic acid copolymer, methacrylic acid/ methyl methacrylate copolymer, methacrylic acid/ ethylacrylate copolymer or methyl acrylate/methacrylic acid/ octyl acrylate copolymer, or methacrylic acid/methylacrylate/methyl methacrylate.

Suitable anionic polymers or copolymers with methacrylic acid as a functional group are sold unter the tradename EUDRAGIT® (Evonik, Germany): EUDRAGIT® L100-55 (sold in powder form; dissolution above pH 5.5), EUDRAGIT® L30 D-55 (sold as aqueous dispersion, 30%; dissolution above pH 5.5), EUDRAGIT® L100 (sold in powder form; dissolution above pH 6.0) or EUDRAGIT® L12,5 (sold as organic solution, 12.5%; dissolution above pH 6.0), EUDRAGIT® S100 (sold in powder form; dissolution above pH 7.0), EUDRAGIT® S12,5 (sold as organic solution, 12.5%; dissolution above pH 7.0) or EUDRAGIT® FS 30D (sold as aqueous dispersion, 30%; dissolution above pH 7.0).

Suitable methacrylate copolymers having neutral ester groups are also sold under the tradename EUDRAGIT® (Evonik, Germany): EUDRAGIT® NE 30D/ 40D /NM 30D and methacrylate copolymers having trimethylammonioethyl groups are sold under the tradename EUDRAGIT® (Evonik, Germany): EUDRAGIT® RL100, EUDRAGIT® RL PO/ RS PO, EUDRAGIT® RL 30D/RS 30D, and EUDRAGIT® RL12,5/ RS12,5.

Preferred enteric maleic copolymers are vinylacetate/ maleic acid anhydride copolymer, styrene/ maleic acid anhydride copolymer, styrene/ maleic acid monoester copolymer, vinylmethylether/ maleic acid anhydride copolymer, ethylene/ maleic acid anhydride copolymer, vinylbutylether/ maleic acid anhydride copolymer, acrylonitrile/ methyl acrylate/ maleic acid anhydride copolymer or butyl acrylate/styrene/ maleic acid anhydride copolymer.

Preferred enteric polyvinyl derivatives are polyvinyl alcohol phthalate, polyvinylacetal phthalate, polyvinyl butylate phthalate or polyvinylacetoacetal phthalate

One possible coating technique for applying a coating composition, notably a drug-containing layer composition, a protective coating composition, or an enteric coating composition, onto a surface of a particle such as e.g. a neutral core, a granule, a dosage formulation or the like is the technique of spray coating. When applying the process of spray coating, the coating composition can e.g. be sprayed onto fluidized particles, e.g. the drug-loaded pellets.

In a preferred embodiment, the respective layers are applied by spray coating.

General principles for applying enteric coatings are known to a person skilled in the art and are for instance described in the Eudragit® Application Guidelines (10th Revised Edition, 06/2008) or elsewhere.

In a further embodiment, the pellet comprises an enteric coating above the one or more protective layers. Thus, it is a further embodiment that the pellet also comprises a protective layer and an enteric layer, in addition to the neutral core and the pharmaceutically active ingredient layer. Such a pellet is also referred to herein as a so-called final pellet (also referred to herein as enteric coated pellet).

In a preferred embodiment, the final pellet comprises a neutral core, a pharmaceutically active ingredient layer, a protective layer, and an enteric layer, with the following composition:
(i) 30 - 50 wt.-%, preferably 35 - 45 wt.-% neutral core,
(ii) 15 - 30 wt.-% , preferably 15 - 25 wt.-%, more preferably 17 - 25 wt.-%, even more preferably 20 - 25 wt.-% omeprazole,
   3 - 6 wt.-%, preferably 3.5 - 5 wt.-% binder, preferably hydroxypropyl methylcellulose,
   0.5 - 2.0 wt.-%, preferably 0.5 - 1.5 wt.-% alkaline reacting compound, preferably magnesium oxide,
   0.5 - 2.0 wt.-%, preferably 0.5 - 1.5 wt.-% surfactant, preferably sodium lauryl sulfate,
   all under (ii) included in a pharmaceutically active ingredient layer,
(iii) 10 - 20 wt.-%, preferably 10 - 18 wt.-% protective layer, preferably comprising talc and povidone, and
(iv) 10 - 40 wt.-%, preferably 13 - 35 wt.-%, more preferably 15 - 30 wt.-% enteric layer, preferably the enteric layer comprises poly(methacrylic acid-co-ethyl acrylate) (Eudragit®) and triethylcitrate.

In a preferred embodiment, the final pellet comprises a neutral core, a pharmaceutically active ingredient layer a protective layer, and an enteric layer, and with the following composition:
(i) 30 - 50 wt.-%, preferably 35 - 45 wt.-% a neutral core,
(ii) 15 - 30 wt.-% , preferably 15 - 25 wt.-%, more preferably 17 - 25 wt.-%, even more preferably 20 - 25 wt.-% omeprazole,
   3 - 6 wt.-%, preferably 3.5 - 5 wt.-% hydroxypropyl methylcellulose,
   0.5 - 2.0 wt.-%, preferably 0.5 - 1.5 wt.-% magnesium oxide,
   0.5 - 2.0 wt.-%, preferably 0.5 - 1.5 wt.-% sodium lauryl sulfate,
   all under (ii) included in a pharmaceutically active ingredient layer,
(iii) 10 - 20 wt.-%, preferably 10 - 18 wt.-% protective layer, comprising talc and povidone, and
(iv) 10 - 40 wt.-%, preferably 13 - 35 wt.-%, more preferably 15 - 30 wt.-% enteric layer, the enteric layer comprises poly(methacrylic acid-co-ethyl acrylate) (Eudragit®) and triethylcitrate.

The present invention also refers to the use of the pellets as disclosed herein for preparing a pharmaceutical composition. This pharmaceutical composition can additionally comprise one or more further pharmaceutically acceptable excipients. These pharmaceutically acceptable excipients are preferably selected from the group consisting of diluents, binders, fillers, disintegrants, lubricants, controlled release (CR) agents, sweeteners, glidants, flavourings and colouring agents, wherein:
the fillers are selected from the group consisting of different grades of starches, such as maize starch, potato starch, rice starch, wheat starch, pregelatinized starch, fully pregelatinized starch; cellulose, such as microcrystalline cellulose or silicified microcrystalline cellulose; mannitol, erythritol; lactose, such as lactose monohydrate, lactose anhydrous, spray dried lactose or milled lactose; calcium salts, such as calcium hydrogenphosphate; sorbitol, and xylitol; particularly preferably the fillers are selected from the group consisting of pregelatinized starch, microcrystalline cellulose, silicified microcrystalline cellulose, lactose monohydrate, spray dried lactose, and milled lactose;
the disintegrants are selected from the group consisting of carmellose calcium, carboxymethylstarch sodium, croscarmellose sodium (cellulose carboxymethylether sodium salt, crosslinked), starch, and its derivatives such as sodium starch glycolate, crosslinked polyvinylpyrrolidone (crospovidone), and low-substituted hydroxypropylcellulose; particularly preferably the disintegrants are selected from the group consisting of sodium starch glycolate, croscarmellose sodium and crospovidone;
the lubricants are selected from the group consisting of stearic acid, talc, glyceryl behenate, sodium stearyl fumarate and magnesium stearate; particularly preferably the lubricant are magnesium stearate and sodium stearyl fumarate;
the binders are selected from the group consisting of polyvinyl pyrrolidone (Povidone), copolymers of vinylpyrrolidone with other vinylderivatives (Copovidone), hydroxypropyl methylcellulose, methylcellulose, hydroxypropylcellulose, powdered acacia, gelatin, guar gum, carbomer such as carbopol, polymethacrylates and pregelatinized starch;
the diluents are selected from carbohydrates such as monosaccharides like glucose, oligosaccharides like sucrose, anhydrous lactose, lactose monohydrate or milled lactose, and sugar alcohols like sorbitol, mannitol, erythrol, and xylitol; particularly preferably the diluent is selected from the group consisting of sorbitol and milled lactose;
the glidants are selected from the group consisting of colloidal silica, hydrophobic colloidal silica and magnesium trisilicate, such as talc; particularly preferably the glidants are selected from the group consisting of colloidal silica and hydrophobic colloidal silica;
the controlled release (CR) agents for the production of matrix based CR solid dosage forms are preferably selected from the group consisting of high viscosity water soluble polymers such as hydroxypropyl cellulose and hypromellose, and lypophilic matrix forming agents such as glyceryl behenate; and/or
the sweeteners are selected from the group consisting of aspartame, saccharin sodium, dipotassium glycyrrhizinate, aspartame, stevia, thaumatin, and the like; preferably the excipients are microcrystalline cellulose, silicified microcrystalline cellulose, milled lactose, spray dried lactose, croscarmellose sodium, sodium starch glycolate, low substituted hydroxypropylcellulose, crospovidone, magnesium stearate, and sodium stearyl fumarate.

The present invention further refers to a dosage from that comprises a pellet as described herein. Preferably, the dosage from represents a solid oral dosage form, preferably the dosage form is a tablet or a capsule. In a preferred embodiment, the dosage from is a hard capsule.

In a preferred embodiment, the dosage form of the present invention comprises a dose of pharmaceutically active ingredients in the range of from 10 mg to 40 mg, e.g. 10 mg, 20 mg or 40 mg, preferably the dose of pharmaceutically active ingredients in the dosage form is 40 mg.

In a preferred embodiment, capsules, preferably hard capsules, comprising omeprazole have the following sizes:

**Table 2: Capsule sizes**

| Omeprazole strength | Capsule size number | Capsule length | Capsule width |
|---|---|---|---|
| 10 mg | 4 | 14.3 mm | 5.3 mm |
| 20 mg | 4 or 3 | 14.3 mm or 15.9 mm | 5.3 mm or 5.8 mm |
| 40 mg | 2 | 18.0 mm | 6.3 mm |

Finally, the present invention refers to the dosage form of the present invention for use in a method of treating a disease selected from the group consisting of gastro esophageal reflux disease, peptic ulcer disease, and Zollinger-Ellison syndrome and for prevention of upper gastrointestinal bleeding in people who are at high risk.

### Methods

### Method for determining particle size:

The following method can be used for determining the mean particle size, e.g. the mean particle size of the neutral core used in the present invention: European Pharmaocopeia, 7th Edition 2011 (7.2), Chapter 2.9.12: "Sieve analysis", or European Pharmaocopeia, 7th Edition 2011 (7.2), Chapter 2.9.31: Particle size analysis by laser light diffraction using Malvern Mastersizer 2000 and a dry dispersion system

### Description of the figures

**Fig. 1****:** This figure shows common general knowledge about the impact of binder on drug release. In particular, Fig. 1 depicts the time to 90 % drug release from compressed tablets containing the pharmaceutically active ingredient niacinamide and prepared using roller compaction at 3 tons pressure. Fig. 1 is taken from "Wet and Dry granulation Binder - A Technical Review by Dow".
**Fig. 2****:** This figure shows the dissolution of enteric coated pellets of omeprazole in 900 mL phosphate buffer, pH 6.8 after 2 hrs in 0.1 M HCI; Apparatus 1, 100 rpm (USP).
   The applied dissolution method is designed to mimic gastrointestinal environment where the dosage form first reaches the stomach where acidic environment is present and then, after one to two hours, the dosage form passes to duodenum and upper intestinal tract where pH conditional are around pH 7.
   First stage is 0.1 M HCI (pH 1) where gastric resistance is challenged and immediate release part of formulation is dissolved. At second stage phosphate buffer, pH 6.8, is applied.

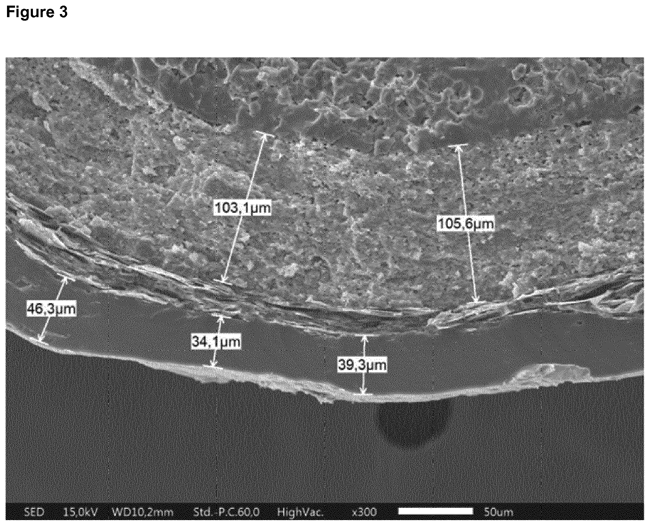

**Fig. 3****:** This figure shows an SEM picture of a relatively high pharmaceutically active ingredient loaded pellet according to the present invention, showing coating thickness of approximately 100 µm for active layer and 40 µm for enteric layer.

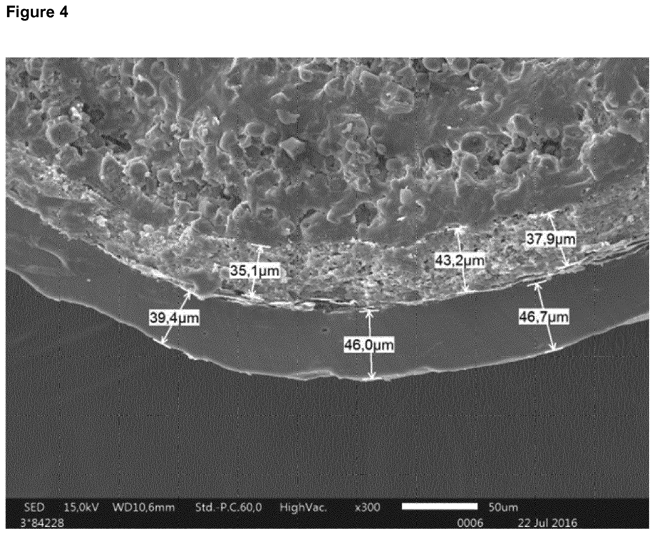

**Fig. 4****:** This figure shows an SEM picture of pellets prepared according to comparative example 2 showing coating thickness of normal loading with approximate less than 40 µm for active layer and 40 µm for enteric layer.

### Examples:

In order to illustrate the present invention, pellets comprising an active layer have been prepared. Examples 1, 2, 3 and 4 are according to the present invention, and Comparative Examples 1 to 6 represent comparative examples. The prepared pellets exhibit the following thickness of active layer (Table 3) and the following API (omeprazole):neutral core ratios, and API (omeprazole):binder ratios (Table 4):

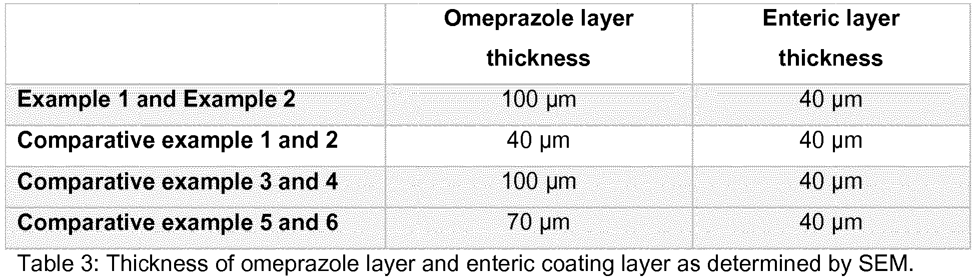

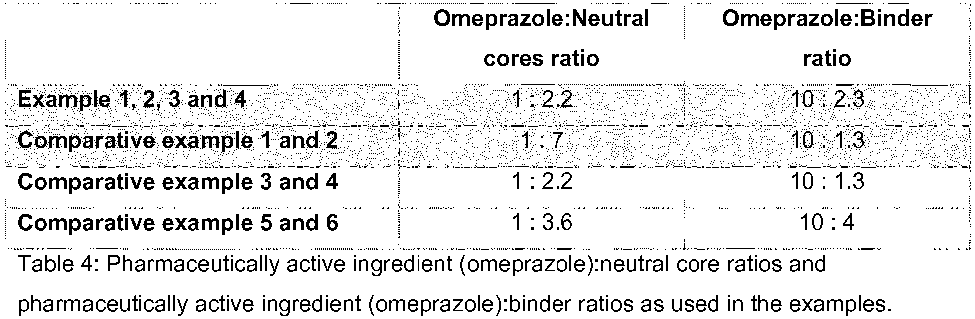

### Example 1:

Preparation of omeprazole-loaded pellets comprising neutral core and omeprazole layer wherein high omeprazole loading and higher amount of hypromellose was used.

| Substance | | Amount per capsule (mg) |
|---|---|---|
| Omeprazole | | 40.0 |
| Neutral sugar cores | | 86.7 |
| Hypromellose (HPMC) | (binder) | 9.0 |
| *Sodium lauryl sulfate* | *(Surfactant)* | *1.5* |
| *Magnesium oxide* | *(Alkalizing agent)* | *2.0* |
| Total mass (mg) | | 139.2 |

| | | |
|---|---|---|
| Omeprazole/neutral cores ratio = 1:2.2 | | |

Omeprazole, Hypromellose (hydroxypropylmethylcellulose; HPMC), Sodium lauryl sulfate and magnesium oxide was dispersed in water using propeller mixer. Neutral sugar cores were loaded into fluid bed apparatus (Huettlin HKC 200). First, neutral cores were heated to 40°C by hot air and then they were coated with prepared omeprazole dispersion. Finally, coated pellets were sieved to remove any fines or agglomerates.

Presented composition can be proportionally adjusted also for 10 and 20 mg doses.

### Example 2:

Preparation of final pellets that in addition to the omeprazole layer further comprise a protective coating (layer) and an enteric coating (layer).

| Substance | Amount per capsule (mg) |
|---|---|
| Omeprazole loaded pellet from example 1 | 139.2 |
| Protective layer | 30.0 |
| Enteric layer | 30.8 |
| Total mass (mg) | 200.0 |

Protective coating was prepared by mixing talc and Povidone in water and enteric coating was prepared by mixing Eudragit L 30 D 55 and triethylcitrate. Omeprazole loaded pellets of example 1 were coated first with protective coat, followed by enteric coat. Finally coated pellets were sieved to remove any fines or agglomerates.

Presented composition can be proportionally adjusted also for 10 and 20 mg doses.

Fig. 2 shows the dissolution profile of enteric coated pellets of Example 2. Fig. 3 shows an SEM picture of the final pellet prepared as described in Example 2, from which the coating thickness of the omeprazole layer can be easily measured.

### Example 3:

Preparation of final pellets that in addition to the omeprazole layer further comprise a protective coating (layer) and an enteric coating (layer).

| Substance | Amount per capsule (mg) |
|---|---|
| Omeprazole loaded pellet from example 1 | 139.2 |
| Protective layer | 30.0 |
| Enteric layer | 35.0 |
| Total mass (mg) | 204.2 |

Protective coating was prepared by mixing talc and Povidone in water and enteric coating was prepared by mixing Eudragit L 30 D 55 and triethylcitrate. Omeprazole loaded pellets of example 1 were coated first with protective coat, followed by enteric coat. Finally coated pellets were sieved to remove any fines or agglomerates.

Presented composition can be proportionally adjusted also for 10 and 20 mg doses.

### Example 4:

Preparation of final pellets that in addition to the omeprazole layer further comprise a protective coating (layer) and an enteric coating (layer).

| Substance | Amount per capsule (mg) |
|---|---|
| Omeprazole loaded pellet from example 1 | 139.2 |
| Protective layer | 30.0 |
| Enteric layer | 70.0 |
| Total mass (mg) | 239.2 |

Protective coating was prepared by mixing talc and Povidone in water and enteric coating was prepared by mixing Eudragit L 30 D 55 and triethylcitrate. Omeprazole loaded pellets of example 1 were coated first with protective coat, followed by enteric coat. Finally coated pellets were sieved to remove any fines or agglomerates.

Presented composition can be proportionally adjusted also for 10 and 20 mg doses.

### Comparative Example 1:

Preparation of standard omeprazole-loaded pellets comprising neutral core and omeprazole layer, wherein relatively low omeprazole loading and relatively low amount of hypromellose was used.

| Substance | | Amount per capsule (mg) |
|---|---|---|
| Omeprazole | | 40.0 |
| Neutral sugar cores | | 280.0 |
| Hypromellose | (Binder) | 5.0 |
| Sodium lauryl sulfate | (Surfactant) | 1.5 |
| Magnesium oxide | (Alkalizing agent) | 2.0 |
| Total mass (mg) | | 328.5 |

| | | |
|---|---|---|
| Omeprazole/Neutral cores ratio = 1:7 | | |

Omeprazole, Hypromellose, sodium lauryl sulfate and magnesium oxide were dispersed in water using propeller mixer. Neutral sugar cores were loaded into fluid bed apparatus (Huettlin HKC 5). First, neutral cores were heated to 40°C by hot air and then they were coated with prepared omeprazole dispersion. Finally, coated pellets were sieved to remove any fines or agglomerates.

Presented composition can be proportionally adjusted also for 10 and 20 mg doses.

### Comparative Example 2:

Preparation of final pellets that in addition to the omeprazole layer further comprise a protective coating (layer) and an enteric coating (layer).

| Substance | Amount per capsule (mg) |
|---|---|
| Omeprazole loaded pellet from comparative example 1 | 328.5 |
| Protective layer | 30.0 |
| Enteric layer | 85 |
| Total mass (mg) | 443.5 |

Protective coating was prepared by mixing talc and Povidone in water and enteric coating was prepared by mixing Eudragit L 30 D 55 and triethylcitrate. Omeprazole loaded pellets of comparative example 1 were coated first with protective coat, followed by enteric coat. Finally coated pellets were sieved to remove any fines or agglomerates.

Presented composition can be proportionally adjusted also for 10 and 20 mg doses.

Fig. 2 shows the dissolution profile of enteric coated pellets of comparative example 2. Fig. 4 shows an SEM picture of the final pellet prepared as described in comparative example 2.

### Comparative Example 3:

Preparation of omeprazole-loaded pellets comprising neutral core and omeprazole layer, wherein relatively high omeprazole loading and relatively low amount of hypromellose was used.

| Substance | | Amount per capsule (mg) |
|---|---|---|
| Omeprazole | | 40.0 |
| Neutral sugar cores | | 86.7 |
| Hypromellose | (binder) | 5.0 |
| *Sodium lauryl sulfate* | *(Surfactant)* | *1.5* |
| *Magnesium oxide* | *(Alkalizing agent)* | *2.0* |
| Total mass (mg) | | 134.2 |

| | | |
|---|---|---|
| Omeprazole/Neutral cores ratio = 1:2.2 | | |

Omeprazole, Hypromellose, sodium lauryl sulfate and magnesium oxide were dispersed in water using propeller mixer. Neutral sugar cores were loaded into fluid bed apparatus (Huettlin HKC 5). First, neutral cores were heated to 40°C by hot air and then they were coated with the prepared omeprazole dispersion. Finally, the coated pellets were sieved to remove any fines or agglomerates.

Presented composition can be proportionally adjusted also for 10 and 20 mg doses.

### Comparative Example 4:

Preparation of final pellets that in addition to the omeprazole layer further comprise a protective coating (layer) and an enteric coating (layer).

| Substance | Amount per capsule (mg) |
|---|---|
| Omeprazole loaded pellet from comparative example 3 | 134.2 |
| Protective layer | 30.0 |
| Enteric layer | 30.8 |
| Total mass (mg) | 195.0 |

Protective coating was prepared by mixing talc and Povidone (polyvinylpyrrolidone) in water, and enteric coating was prepared by mixing Eudragit L 30 D 55 and triethylcitrate (TEC). Omeprazole loaded pellets of comparative example 3 were coated first with protective coat, followed by coating with the enteric coat. Finally, the coated pellets were sieved to remove any fines or agglomerates.

Presented composition can be proportionally adjusted also for 10 and 20 mg doses.

Fig. 2 shows the dissolution profile of enteric coated pellets of comparative example 4.

### Comparative Example 5:

Preparation of omeprazole-loaded pellets comprising neutral core and omeprazole layer, wherein medium omeprazole loading and very high amount of hypromellose was used.

| Substance | | Amount per capsule (mg) |
|---|---|---|
| Omeprazole | | 40.0 |
| Neutral sugar cores | | 143 |
| Hypromellose | | 16.0 |
| *Sodium lauryl sulfate* | *(Surfactant)* | *1.5* |
| *Magnesium oxide* | *(Alkalizing agent)* | *2.0* |
| Total mass (mg) | | 202.50 |

| | | |
|---|---|---|
| Omeprazole/Neutral cores ratio = 1:3.6 | | |

Omeprazole, Hypromellose, sodium lauryl sulfate and magnesium oxide were dispersed in water using propeller mixer. Neutral sugar cores were loaded into fluid bed apparatus (Huettlin HKC 5). First, neutral cores were heated to 40°C by hot air and then they were coated with prepared omeprazole dispersion. Finally, coated pellets were sieved to remove any fines or agglomerates.

Presented composition can be proportionally adjusted also for 10 and 20 mg doses.

### Comparative Example 6:

Preparation of final pellets that in addition to the omeprazole layer further comprise a protective coating (layer) and an enteric coating (layer).

| Substance | Amount per capsule (mg) |
|---|---|
| Omeprazole loaded pellet from comparative example 5 | 202.5 |
| Protective layer | 30.0 |
| Enteric layer | 52.5 |
| Total mass (mg) | 285.0 |

Protective coating was prepared by mixing talc and Povidone in water and enteric coating was prepared by mixing Eudragit L 30 D 55 and triethylcitrate. Omeprazole loaded pellets of comparative example 5 were coated first with protective coat, followed by enteric coat. Finally coated pellets were sieved to remove any fines or agglomerates.

Presented composition can be proportionally adjusted also for 10 and 20 mg doses.

Fig. 2 shows the dissolution profile of enteric coated pellets of comparative example 6.

### Increase in batch size in number of produced pieces

As is shown in the following table (Table 5), by applying the present invention the manufacturing procedure can be improved. This is due to increasing the omeprazole loading on neutral cores, thus increasing the omeprazole layer on said cores. This, in turn, is achieved by reducing the amount of excipients and increasing the batch size in number of produced capsules produced on the same equipment. This significantly improves manufacturing effectiveness. As is shown in the table below (Table 5), a significant increase in batch size in terms of produced pieces is possible for the same equipment (batch size in kg).

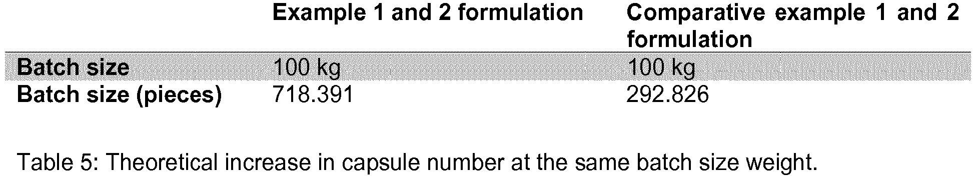

### Binder impact on formation of agglomerates

The binder has an important role in binding the API on the neutral cores. Thus, it can also bind together two or more pellets which however is not desired as such agglomerates are rejected due to the quality characteristics. Table 6 below shows the portion of agglomerates that is present when using different amounts (ratios) of binder.

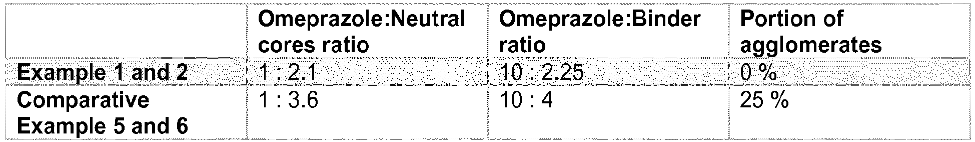

## Claims

1. Pellet comprising
- a neutral core comprising one or more pharmaceutically acceptable excipients,
- a pharmaceutically active ingredient layer surrounding the neutral core, wherein said pharmaceutically active ingredient layer comprises omeprazole, one or more binders, and optionally one or more further excipients, wherein the thickness of said pharmaceutically active ingredient layer is more than 50 µm at one or more positions; and wherein the weight ratio of omeprazole:binder(s) is in the range of from 3:1 to 5:1 and
- wherein the weight ratio of omeprazole:neutral core is in the range of from 1:1.5 to 1:4.

2. The pellet according to claim 1, wherein the neutral core comprises of one or more compounds selected from the group consisting of inorganic salts, metal oxides, organic polymers, such as celluloses, starches and sugars, preferably the neutral core is approximately spherical.

3. The pellet according to any of the preceding claims, wherein the thickness of the pharmaceutically active ingredient layer is preferably in a range of from 60 µm to 150 µm, more preferably from 70 µm to 140 µm, and even more preferably from 80 µm to 120 µm at one or more positions.

4. The pellet according to any of the preceding claims, wherein the binder content in the drug-containing layer is at least 15 wt.-% and/or wherein the pharmaceutically active ingredient content in the drug-containing layer is at least 65 wt.-%.

5. The pellet according to any of the preceding claims, wherein the pharmaceutically active ingredient layer comprises one or more further excipients selected from the group consisting of alkaline reacting substances and surfactants.

6. The pellet according to any of the preceding claims, wherein the binder is selected from the group consisting of celluloses, such as hydroxypropyl methylcellulose, hydroxypropyl cellulose and carboxymethyl-cellulose sodium; polyvinyl pyrrolidone; sugars; and starches; preferably the binder is hydroxypropylmethylcellulose.

7. The pellet according to any of the preceding claims, comprising a neutral core and pharmaceutically active ingredient layer with the following composition:
(i) 50 - 70 wt.-%, preferably 55 - 65 wt.-%, more preferably 60 - 65 wt.-% neutral core, and,
(ii) 20 - 40 wt.-%, preferably 25 - 35 wt.-percent, more preferably 25 - 30 wt.-% omeprazole,
5 - 8 wt.-%, preferably 6 - 7 wt.-% binder, preferably hydroxypropyl methylcellulose,
0.5 - 2.0 wt.-%, preferably 0.5 - 1.5 wt.-% alkaline reacting compound, preferably magnesium oxide,
0.5 - 2.0 wt.-%, preferably 0.5 - 1.5 wt.-% surfactant, preferably sodium lauryl sulfate,
all under (ii) included in a pharmaceutically active ingredient layer.

8. The pellet according to any of the preceding claims, comprising a neutral core, a pharmaceutically active ingredient layer, a protective layer, and an enteric layer, with the following composition:
(i) 30 - 50 wt.-%, preferably 35 - 45 wt.-% neutral core,
(ii) 15- 30 wt.-% , preferably 17 - 30 wt.-%, more preferably 17 - 25 wt.-%, preferably 20 - 25 wt.-% omeprazole,
3 - 6 wt.-%, preferably 3.5 - 5 wt.-% binder, preferably hydroxypropyl methylcellulose,
0.5 - 2.0 wt.-%, preferably 0.5 - 1.5 wt.-% alkaline reacting compound, preferably magnesium oxide,
0.5 - 2.0 wt.-%, preferably 0.5 - 1.5 wt.-% surfactant, preferably sodium lauryl sulfate,
all under (ii) included in a pharmaceutically active ingredient layer,
(iii) 10 - 20 wt.-%, preferably 10 - 18 wt.-% protective layer, preferably comprising talc and povidone, and
(iv) 10 - 40 wt.-%, preferably 13 - 35 wt.-%, more preferably 15 - 30 wt.-% enteric layer, preferably the enteric layer comprises poly(methacrylic acid-co-ethyl acrylate) and triethylcitrate.

9. Process for preparing a pellet according to any of claims 1 to 8, wherein the pharmaceutically active ingredient layer is applied by a layering process using a fluidized bed spray granulator, wherein a suspension or solution comprising omeprazole, the binder and the optional further excipients is sprayed onto the neutral core and dried.

10. Use of the pellet of any of claims 1 to 8 for preparing a pharmaceutical composition.

11. Pharmaceutical composition comprising pellets of any of claims 1 to 8, and optionally further pharmaceutically acceptable excipients.

12. Dosage form, preferably a hard capsule, comprising a pellet of any of claims 1 to 8, or a pharmaceutical composition of claim 11.

13. The hard capsule according to claim 12, wherein the dose of omeprazole in the capsule is in the range of from 10 mg to 40 mg, preferably 10 mg or 20 mg or 40 mg.

14. The hard capsule according to claim 13,
wherein the the dose of omeprazole is 10 mg and the capsule has a length of about 14.3 mm and a width of about 5.3 mm;
or wherein the the dose of omeprazole is 20 mg and the capsule has a length of about 15.9 mm and a width of about 5.8 mm;
or wherein the the dose of omeprazole is 40 mg and the capsule has a length of about 18.0 mm and a width of about 6.3 mm.

15. The dosage form of any one of proceeding claims 12 to 14 for use in a method of treating a disease selected from the group consisting of gastro esophageal reflux disease, peptic ulcer disease, and Zollinger-Ellison syndrome and for prevention of upper gastrointestinal bleeding in people who are at high risk.

## Patentansprüche

1. Pellet umfassend
- einen neutralen Kern umfassend einen oder mehrere pharmazeutisch akzeptable Hilfsstoffe,
- eine Schicht mit pharmazeutischem Wirkstoff, die den neutralen Kern umgibt, wobei die Schicht mit pharmazeutischem Wirkstoff Omeprazol umfasst, ein oder mehrere Bindemittel, und optional einen oder mehrere weitere Hilfsstoffe, wobei die Dicke der Schicht mit pharmazeutischem Wirkstoff mehr als 50 µm an einer oder mehreren Positionen beträgt; und wobei das Gewichtsverhältnis von Omeprazol : Bindemittel(n) im Bereich von 3:1 bis 5:1 liegt, und
- wobei das Gewichtsverhältnis von Omeprazol : neutralem Kern im Bereich von 1:1,5 bis 1:4 liegt.

2. Pellet nach Anspruch 1, wobei der neutrale Kern eine oder mehrere Verbindungen umfasst, ausgewählt aus der Gruppe bestehend aus anorganischen Salzen, Metalloxiden, organischen Polymeren, wie Cellulosen, Stärken und Zuckern, wobei der neutrale Kern bevorzugt ungefähr kugelförmig ist.

3. Pellet nach irgendeinem der vorhergehenden Ansprüche, wobei die Dicke der Schicht mit pharmazeutischem Wirkstoff bevorzugt in einem Bereich von 60 µm bis 150 µm, mehr bevorzugt von 70 µm bis 140 µm, und noch stärker bevorzugt von 80 µm bis 120 µm an einer oder mehreren Positionen beträgt.

4. Pellet gemäß irgendeinem der vorhergehenden Ansprüche, wobei der Gehalt an Bindemittel in der Wirkstoff-enthaltenden Schicht mindestens 15 Gew.-% beträgt und/oder wobei der Gehalt an pharmazeutisch aktivem Wirkstoff in der Wirkstoff-enthaltenen Schicht mindestens 65 Gew.-% beträgt.

5. Pellet gemäß irgendeinem der vorhergehenden Ansprüche, wobei die Schicht mit pharmazeutischem Wirkstoff einen oder mehrere weitere Hilfsstoffe ausgewählt aus der Gruppe bestehend aus alkalisch reagierenden Substanzen und Tensiden umfasst.

6. Pellet gemäß irgendeinem der vorhergehenden Ansprüche, wobei das Bindemittel ausgewählt ist aus der Gruppe bestehend aus Cellulosen, wie Hydroxypropylmethylcellulose, Hydroxypropylcellulose und NatriumCarboxymethylcellulose; Polyvinylpyrrolidon; Zuckern, und Stärken; bevorzugt ist das Bindemittel Hydroxypropylmethylcellulose.

7. Pellet gemäß irgendeinem der vorhergehenden Ansprüche, umfassend einen neutralen Kern und eine Schicht mit pharmazeutischem Wirkstoff mit der folgenden Zusammensetzung:
(i) 50-70 Gew.-%, bevorzugt 55-65 Gew.-%, mehr bevorzugt 60-65 Gew.-% neutraler Kern, und,
(ii) 20-40 Gew.-%, bevorzugt 25-35 Gew.-%, mehr bevorzugt 25-30 Gew.-% Omeprazol,
5-8 Gew.-%, bevorzugt 6-7 Gew.-% Bindemittel, bevorzugt Hydroxypropylmethylcellulose,
0,5-2,0 Gew.-%, bevorzugt 0,5-1,5 Gew.-% alkalisch reagierende Verbindung, bevorzugt Magnesiumoxid,
0,5-2,0 Gew.-%, bevorzugt 0,5-1,5 Gew.-% Tensid, vorzugsweise Natriumlaurylsulfat,
alle unter (ii) in einer Schicht mit pharmazeutischem Wirkstoff eingeschlossen.

8. Pellet gemäß irgendeinem der vorhergehenden Ansprüche, umfassend einen neutralen Kern, eine Schicht mit pharmazeutischem Wirkstoff, eine Schutzschicht und eine enterische Schicht, mit der folgenden Zusammensetzung:
(i) 30-50 Gew.-%, bevorzugt 35-45 Gew.-% neutraler Kern,
(ii) 15-30 Gew.-%, bevorzugt 17-30 Gew.-%, mehr bevorzugt 17-25 Gew.-%, bevorzugt 20-25 Gew.-% Omeprazol,
3-6 Gew.-%, bevorzugt 3,5-5 Gew.-% Bindemittel, bevorzugt Hydroxypropylmethylcellulose,
0,5-2,0 Gew.-%, bevorzugt 0,5-1,5 Gew.-% alkalisch reagierende Verbindung, bevorzugt Magnesiumoxid,
0,5-2,0 Gew.-%, bevorzugt 0,5-1,5 Gew.-% Tensid, bevorzugt Natriumlaurylsulfat,
alle unter (ii) in einer Schicht mit pharmazeutischem Wirkstoff eingeschlossen,
(iii) 10-20 Gew.-%, bevorzugt 10-18 Gew.-% Schutzschicht, bevorzugt umfassend Talkum und Povidon, und
(iv) 10-40 Gew.-%, bevorzugt 13-35 Gew.-%, mehr bevorzugt 15-30 Gew.-% enterische Schicht, bevorzugt umfasst die enterische Schicht Poly(methacrylsäure-Co-Ethylacrylat) und Triethylcitrat.

9. Verfahren zur Herstellung eines Pellets gemäß irgendeinem der Ansprüche 1 bis 8, wobei die Schicht mit pharmazeutischem Wirkstoff durch ein Schichtverfahren unter Verwendung eines Wirbelschicht-Sprühgranulators aufgebracht wird, wobei eine Suspension oder Lösung enthaltend Omeprazol, das Bindemittel und die optional weiteren Hilfsstoffe, auf den neutralen Kern gesprüht und getrocknet wird.

10. Verwenden des Pellets gemäß irgendeinem der Ansprüche 1 bis 8 zur Herstellung einer pharmazeutischen Zusammensetzung.

11. Pharmazeutische Zusammensetzung umfassend Pellets gemäß irgendeinem der Ansprüche 1 bis 8, und optional weitere pharmazeutisch akzeptable Hilfsstoffe.

12. Darreichungsform, bevorzugt eine Hartkapsel, umfassend ein Pellet gemäß irgendeinem der Ansprüche 1-8, oder eine pharmazeutische Zusammensetzung gemäß Anspruch 11.

13. Hartkapsel gemäß Anspruch 12, wobei die Omeprazol-Dosis in der Kapsel im Bereich von 10 mg bis 40 mg, bevorzugt 10 mg oder 20 mg oder 40 mg, liegt.

14. Hartkapsel gemäß Anspruch 13, wobei die Omeprazol-Dosis 10 mg beträgt und die Kapsel eine Länge von ungefähr 14,3 mm und eine Breite von ungefähr 5,3 mm aufweist;
oder wobei die Omeprazol-Dosis 20 mg beträgt und die Kapsel eine Länge von ungefähr 15,9 mm und eine Breite von ungefähr 5,8 mm aufweist;
oder wobei die Omeprazol-Dosis 40 mg beträgt und die Kapsel eine Länge von ungefähr 18,0 mm und eine Breite von ungefähr 6,3 mm aufweist.

15. Darreichungsform gemäß irgendeinem der vorhergehenden Ansprüche 12 bis 14 zur Verwendung in einem Verfahren der Behandlung einer Krankheit ausgewählt aus der Gruppe bestehend aus gastroösophagealer Reflux-Krankheit, Magengeschwür-Erkrankung und Zollinger-Allison-Syndrom und zur Vorbeugung von oberen gastrointestinalen Blutungen bei Menschen mit hohem Risiko.

## Revendications

1. Gélule comprenant
- un noyau neutre comprenant un ou plusieurs excipients pharmaceutiquement acceptables,
- une couche de principe pharmaceutiquement actif entourant le noyau neutre, dans laquelle ladite couche de principe pharmaceutiquement actif comprend de l'oméprazole, un ou plusieurs liants, et facultativement un ou plusieurs excipients supplémentaires, dans laquelle l'épaisseur de ladite couche de principe pharmaceutiquement actif fait plus de 50 µm à une ou plusieurs positions ;
et dans laquelle le rapport de poids de l'oméprazole sur le(s) liant(s) est dans la gamme de 3:1 à 5:1 et
- dans laquelle le rapport de poids de l'oméprazole sur le noyau neutre est dans la gamme de 1:1,5 à 1:4.

2. Gélule selon la revendication 1, dans laquelle le noyau neutre comprend un ou plusieurs composés sélectionnés à partir du groupe constitué par des sels inorganiques, des oxydes métalliques, des polymères organiques, tels que des celluloses, des amidons et des sucres, de préférence le noyau neutre est approximativement sphérique.

3. Gélule selon l'une quelconque des revendications précédentes, dans laquelle l'épaisseur de la couche de principe pharmaceutiquement actif est de préférence dans une gamme de 60 µm à 150 µm, plus préférentiellement de 70 µm à 140 µm, et encore plus préférentiellement de 80 µm à 120 µm à une ou plusieurs positions.

4. Gélule selon l'une quelconque des revendications précédentes, dans laquelle la teneur en liant dans la couche contenant le médicament est au moins de 15 % en poids et/ou dans laquelle la teneur en principe pharmaceutiquement actif dans la couche contenant le médicament est au moins de 65 % en poids.

5. Gélule selon l'une quelconque des revendications précédentes, dans laquelle la couche de principe pharmaceutiquement actif comprend un ou plusieurs excipients supplémentaires sélectionnés à partir du groupe constitué par des substances et des tensioactifs à réaction alcaline.

6. Gélule selon l'une quelconque des revendications précédentes, dans laquelle le liant est sélectionné à partir du groupe constitué par des celluloses, tels que l'hydroxypropylméthylcellulose, l'hydroxypropylcellulose et la carboxyméthylcellulose sodique ; la polyvinylpyrrolidone, des sucres ; et des amidons ; de préférence le liant est l'hydroxypropylméthylcellulose.

7. Gélule selon l'une quelconque des revendications précédentes, comprenant un noyau neutre et une couche de principe pharmaceutiquement actif avec la composition suivante :
(i) de 50 à 70 % en poids, de préférence de 55 à 65 % en poids, plus préférentiellement de 60 à 65 % en poids de noyau neutre, et,
(ii) de 20 à 40 % en poids, de préférence de 25 à 35 % en poids, plus préférentiellement de 25 à 30 % en poids d'oméprazole,
de 5 à 8 % en poids, de préférence de 6 à 7 % en poids de liant, de préférence d'hydroxypropylméthylcellulose,
de 0,5 à 2,0 % en poids, de préférence de 0,5 à 1,5 % en poids de composé à réaction alcaline, de préférence d'oxyde de magnésium,
de 0,5 à 2,0 % en poids, de préférence de 0,5 à 1,5 % en poids de tensioactif, de préférence de laurylsulfate de sodium,
tous ceux sous (ii) inclus dans une couche de principe pharmaceutiquement actif.

8. Gélule selon l'une quelconque des revendications précédentes, comprenant un noyau neutre, une couche de principe pharmaceutiquement actif, une couche protectrice et une couche entérique, avec la composition suivante :
(i) de 30 à 50 % en poids, de préférence de 35 à 45 % en poids de noyau neutre,
(ii) de 15 à 30 % en poids, de préférence de 17 à 30 % en poids, plus préférentiellement de 17 à 25 % en poids, de préférence de 20 à 25 % en poids d'oméprazole,
de 3 à 6 % en poids, de préférence de 3,5 à 5 % en poids de liant, de préférence d'hydroxypropylméthylcellulose,
de 0,5 à 2,0 % en poids, de préférence de 0,5 à 1,5 % en poids de composé à réaction alcaline, de préférence d'oxyde de magnésium,
de 0,5 à 2,0 % en poids, de préférence de 0,5 à 1,5 % en poids de tensioactif, de préférence de laurylsulfate de sodium,
tous ceux sous (ii) inclus dans une couche de principe pharmaceutiquement actif,
(iii) de 10 à 20 % en poids, de préférence de 10 à 18 % en poids de couche protectrice, de préférence comprenant du talc et de la povidone, et
(iv) de 10 à 40 % en poids, de préférence de 13 à 35 % en poids, plus préférentiellement de 15 à 30 % en poids de couche entérique, de préférence la couche entérique comprend du poly(acide méthacrylique-co-acrylate d'éthyle) et du triéthylcitrate.

9. Processus de préparation d'une gélule selon l'une quelconque des revendications 1 à 8, dans lequel la couche de principe pharmaceutiquement actif est appliquée par un processus de stratification en utilisant un granulateur par pulvérisation à lit fluidisé, dans lequel une suspension ou une solution comprenant de l'oméprazole, le liant et les excipients supplémentaires facultatifs est pulvérisée sur le noyau neutre et séchée.

10. Utilisation de la gélule selon l'une quelconque des revendications 1 à 8 pour préparer une composition pharmaceutique.

11. Composition pharmaceutique comprenant des gélules selon l'une quelconque des revendications 1 à 8, et facultativement des excipients pharmaceutiquement acceptables supplémentaires.

12. Forme posologique, de préférence une capsule solide, comprenant une gélule selon l'une quelconque des revendications 1 à 8, ou une composition pharmaceutique selon la revendication 11.

13. Capsule solide selon la revendication 12, dans laquelle la dose d'oméprazole dans la capsule est dans la gamme de 10 mg à 40 mg, de préférence 10 mg ou 20 mg ou 40 mg.

14. Capsule solide selon la revendication 13,
dans laquelle la dose d'oméprazole est de 10 mg et la capsule a une longueur d'environ 14,3 mm et une largeur d'environ 5,3 mm ;
ou dans laquelle la dose d'oméprazole est de 20 mg et la capsule a une longueur d'environ 15,9 mm et une largeur d'environ 5,8 mm ;
ou dans laquelle la dose d'oméprazole est de 40 mg et la capsule a une longueur d'environ 18,0 mm et une largeur d'environ 6,3 mm.

15. Forme posologique selon l'une quelconque des revendications précédentes 12 à 14 pour une utilisation dans un procédé de traitement d'une maladie sélectionnée à partir du groupe constitué par la maladie du reflux gastro-oesophagien, la maladie de l'ulcère gastroduodénal, et le syndrome de Zollinger-Ellison et pour la prévention d'hémorragies de l'appareil gastrointestinal supérieur chez les personnes qui en ont un risque élevé.
